# EUROPEAN PATENT APPLICATION

(11) **EP 4 190 255 A1**
(43) Date of publication of application: **07.06.2023**
(21) Application number: 22209135.7
(22) Date of filing: 23.11.2022
(51) Int. Cl.: A61B 18/02

(54) **METHOD TO MITIGATE BALLOON BREACH DURING CRYOBALLOON THERAPY**

(30) Priority: 01.12.2021 US 202163284688 P; 02.11.2022 US 202218052125
(71) Applicant: Medtronic Cryocath LP, Toronto, Ontario M5L 1B9 (CA)
(72) Inventor: Ma, Wing-Choi, Toronto, M5L 1B9 (CA)
(74) Representative: Zimmermann & Partner Patentanwälte mbB

(57) **Abstract**

A method of predicting an adverse event during an ablation procedure includes providing a medical device having an expandable element and positioning the medical device proximate to an area of target tissue. The medical device includes a fluid exhaust lumen and a fluid supply lumen each being in fluid communication with the expandable element. The method further includes delivering fluid to expandable element and exhausting fluid from the expandable element; measuring a pressure within a vacuum return path; and measuring a period of time it takes for the pressure within the vacuum return path to reach a target pressure.

## Description

### CROSS-REFERENCE TO RELATED APPLICATIONS

The present application is related to and claims benefit under 35 U.S.C. §119(c) to U.S. Provisional Patent Application Serial Number 63/284,688, filed December 1, 2021, entitled "Method to Mitigate Double Balloon Breach During Cryoballoon Therapy," the entire contents of which being incorporated herein by reference.

### FIELD

The present technology is generally related to a device and system, and a related method thereof, of predicting an adverse event during a treatment procedure.

### BACKGROUND

Existing catheter devices used in cryoablation procedures have a double balloon construction with an outer balloon acting as a safety feature to contain the coolant in the event of an inner balloon breach. The space between the inner balloon an outer balloon is under vacuum during cryotherapy, and a console continuously monitors the pressure between the two balloons to detect the case of a balloon breach and to stop the injection or delivery of coolant. However, although a double balloon breach is extremely rare, it is still possible. When a double balloon breach occurs, there is a sudden release of pressurized gas which may be dangerous or harmful to the patient.

### SUMMARY

The techniques of this disclosure generally relate to devices, systems, and methods of use thereof, for predicting an adverse event during a medical procedure.

One aspect provides a method of predicting an adverse event during an ablation procedure includes providing a medical device having an expandable element and positioning the medical device proximate to an area of target tissue. The medical device includes a fluid exhaust lumen and a fluid supply lumen each being in fluid communication with the expandable element. The method further includes delivering fluid to expandable element and exhausting fluid from the expandable element; measuring a pressure within a vacuum return path; and measuring a period of time it takes for the pressure within the vacuum return path to reach a target pressure.

In some aspects, the medical device is in communication with a console. The console includes processing circuitry, a fluid supply reservoir in fluid communication with the fluid supply lumen, and a fluid exhaust chamber in fluid communication with the fluid exhaust lumen.

In some aspects, the method further includes triggering a fault to stop the delivery of fluid if the pressure within the vacuum return path does not reach the target pressure within a predetermined time period.

In some aspects, the vacuum return path is disposed within the console. The pressure of the vacuum return path is measured by a pressure sensor disposed within the console along the vacuum return path.

In some aspects, the method further includes generating an alert when the fault to stop the delivery of fluid is triggered. The alert is at least one of an audible alert, a visual alert, and a tactile alert indicative of a presence of an adverse event.

In some aspects, the adverse event is a condition causing the restriction of fluid through the vacuum return path.

In some aspects, the method further includes measuring, with a flow sensor, a mass flow rate of fluid traveling from the expandable element to the console via the fluid exhaust lumen.

In some aspects, the method further includes comparing the measured mass flow rate of fluid to a mass flow rate threshold, triggering the fault if it is determined that the adverse event is detected based on the comparison of the measured mass flow rate of fluid to the mass flow rate threshold, and generating the alert when the fault to stop the delivery of fluid is triggered.

In some aspects, the medical device further includes a pressure monitoring tube at least partially disposed within the expandable element.

Another aspect provides a medical system includes a console having processing circuitry and a medical device in communication with the console. The medical device includes an expandable element, a fluid supply lumen and a fluid exhaust lumen in fluid communication with the expandable element and the console, and a pressure sensor. The fluid exhaust lumen being in communication with a vacuum return path disposed within the console. The pressure sensor is disposed within the console along the vacuum return path and is configured to measure a pressure within the vacuum return path.

In some aspects, the system further includes a fluid supply reservoir and a fluid exhaust chamber each disposed within the console and in fluid communication with the medical device.

In some aspects, the processing circuitry is configured to measure a period of time it takes for the pressure within the vacuum return path to reach a target pressure.

In some aspects, the processing circuitry is further configured to: initiate a delivery of fluid from the fluid supply reservoir to the expandable element, and trigger a fault to stop the delivery of fluid to the expandable element if the measured pressure within the vacuum return path does not reach the target pressure within a predetermined time period.

In some aspects, the console is configured to generate and transmit an alert when the fault is triggered. The alert is at least one of an audible alert, a visual alert, and a tactile alert indicative of a presence of an adverse event.

In some aspects, a flow sensor is disposed within the console along the vacuum return path. The flow sensor is configured to measure a mass flow rate of fluid traveling from the expandable element to the fluid exhaust chamber via the vacuum return path.

In some aspects, the processing circuitry is configured to compare the measured mass flow rate of fluid to a mass flow rate threshold and trigger a fault to stop the delivery of fluid to the expandable element if the measured mass flow rate of fluid deviates from the mass flow rate threshold by more than a predetermined range.

In some aspects, the medical device further includes a pressure monitoring tube at least partially disposed within the expandable element.

Yet another aspect provides a method of predicting an adverse event during an ablation procedure includes providing a medical device having an expandable element. The medical device is in communication with a console having processing circuitry, a fluid supply reservoir, a fluid exhaust chamber, and a vacuum return path. The method further includes positioning the medical device proximate to an area of target tissue, the medical device includes a fluid exhaust lumen and a fluid supply lumen each being in fluid communication with the expandable element and the console, and delivering fluid from the fluid supply reservoir to expandable element and exhausting fluid from the expandable element to the fluid exhaust chamber. The method further includes at least one selected from the group consisting of: (a) measuring a pressure of the vacuum return path, the pressure being measured by a pressure sensor disposed within the console along the vacuum return path; (b) measuring a period of time it takes for the pressure within the vacuum return path to reach a target pressure; (c) measuring, with a flow sensor, a mass flow rate of fluid traveling from the expandable element to the fluid exhaust chamber via the vacuum return path; and (d) comparing the measured mass flow rate of fluid to a mass flow rate threshold. The method further includes triggering a fault to stop the delivery of fluid to the expandable element if the pressure of the vacuum return path does not reach the target pressure within a predetermined time period or if the measured mass flow rate of fluid deviates from the mass flow rate threshold by more than a predetermined range.

In some aspects, the method further includes generating an alert when the fault is triggered. The alert is at least one of an audible alert, a visual alert, and a tactile alert indicative of a presence of an adverse event.

The details of one or more aspects of the disclosure are set forth in the accompanying drawings and the description below. Other features, objects, and advantages of the techniques described in this disclosure will be apparent from the description and drawings, and from the claims.

### BRIEF DESCRIPTION OF THE DRAWINGS

The accompanying figures, where like reference numerals refer to identical or functionally similar elements throughout the separate views, together with the detailed description below, are incorporated in and form part of the specification, and serve to further illustrate aspects, features, examples, and embodiments of concepts that include the claimed subject matter, and explain various principles and advantages of those aspects, features, examples, and embodiments.
FIG. 1 illustrates an example medical system constructed according to some examples.
FIG. 2 illustrates an example fluid pathway of the medical system of FIG. 1 according to some examples.
FIG. 3 illustrates a difference in measured pressure over time between a kinked catheter and a normal operating catheter according to some examples.
FIG. 4 illustrates a dip in measured mass fluid flow rate from a predetermined mass flow rate threshold during an adverse event according to some examples.
FIG. 5 is a flow chart of an exemplary use of medical system of FIGS. 1-2 according to some examples.

Skilled artisans will appreciate that elements in the figures are illustrated for simplicity and clarity and have not necessarily been drawn to scale. For example, the dimensions of some of the elements in the figures may be exaggerated relative to other elements to help to improve understanding of aspects, features, examples, and embodiments.

The apparatus and method components have been represented where appropriate by conventional symbols in the drawings, showing only those specific details that are pertinent to understanding the aspects, features, examples, and embodiments presented so as not to obscure the disclosure with details that will be readily apparent to those of ordinary skill in the art having the benefit of the description herein.

### DETAILED DESCRIPTION

The devices, systems, and methods disclosed herein are for treating an area of tissue, such as performing pulmonary vein isolation, spot ablation, and/or linear ablation with a single medical device. For example, a system is provided that includes a medical device in communication with a console having a pressure sensor disposed therein.

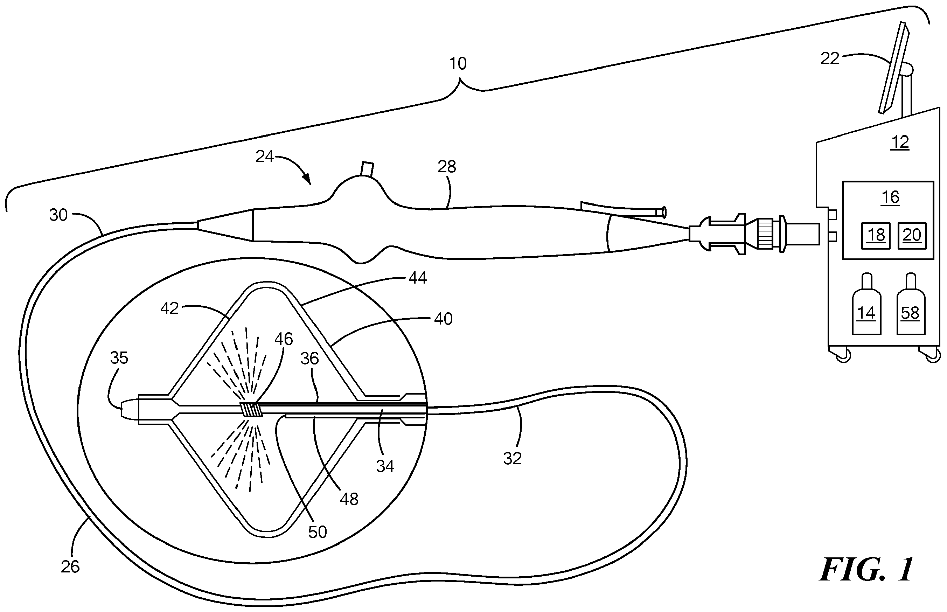

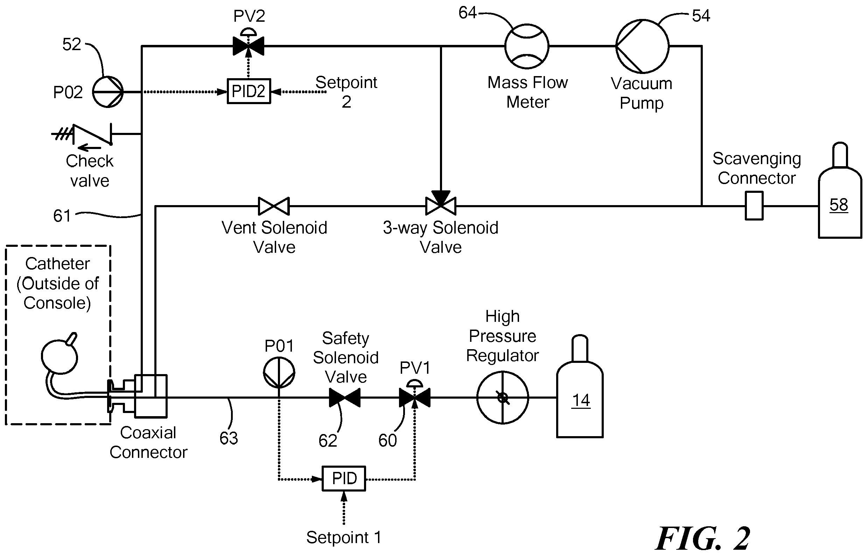

Referring now to the drawings in which like reference designators refer to like elements, there is shown in FIG. 1 an exemplary medical system 10 according to some aspects. The system 10 may include a console 12 configured to control the operation of the various components described herein. The console 12 may include a fluid supply reservoir 14 retained within or otherwise coupled to the console 12. The fluid supply reservoir 14 may be configured to retain a supply of fluid (i.e., liquid, gas, and/or vapor) such as, for example, air, saline, and/or cryogenic fluid (i.e., coolant or refrigerant such as N₂O or argon gas) which may be used to thermally treat a target tissue region (for example, bronchial or gallbladder tissue). Coupled to the fluid supply reservoir 14 may be one or more valves and/or regulators (shown in FIG. 2) configured to control the flow of fluid from the fluid supply reservoir 14. In some embodiments, the one or more valves may be a check valve or other one-way valves. The console 12 includes processing circuitry 16 having a memory 18 and a processor 20 configured to carry out the various functions of the console 12. The console 12 may further include a display 22 configured to display the various functions and operations of the console 12 before, during, and after application of thermal energy.

The system 10 may further include a medical or treatment device 24 configured to fluidly and electrically couple to the console 12 through one or more umbilicals (not shown). The medical device 24 may be a cryogenic catheter configured to extract heat from a target tissue region by delivering cryogenic fluid to a treatment element (for example, a cryoballoon or other similar expandable element) that comes into physical contact with target tissue during an ablation procedure. The medical device 24 may include an elongate body 26 coupled to a handle 28 of the medical device 24. The elongate body 26 has a proximal portion 30 and a distal portion 32 opposite the proximal end, one or more lumens therein configured transport fluid from the fluid supply reservoir 14 through the medical device 24 and back out through the medical device 24. For example, the medical device 24 may include a supply lumen 36 configured to transport fluid into the medical device 24 and an exhaust lumen (not shown) configured to transport fluid away from the medical device 24. Also, the medical device 24 may include an inner shaft 34 coupled to the handle 28 and extending through the elongate body 26 such that a proximal portion (not shown) of the inner shaft 34 is proximate to the proximal portion 30 and a distal portion of the inner shaft 34 is proximate to or extends out of an opening defined by the distal portion 32. Although not shown in detail herein, it is to be understood that in some configurations, the exhaust lumen is defined between the inner surface of the elongate body 26 and the outer surface of the inner shaft 34.

As shown in FIG. 1, the medical device 24 further includes an expandable element 40 coupled to the inner shaft 34. In some embodiments, the expandable element 40 is an inflatable balloon sized and is configured to surround at least a portion of the inner shaft 34 and contact a wall of the target tissue region when inflated. The inner shaft 34 may extend through the expandable element 40 and define a distal tip 35 that extends distally beyond the expandable element 40. In an exemplary configuration, the expandable element 40 includes an inner surface and an outer surface, the inner surface being in fluid communication with the fluid when fluid is supplied to the balloon 40 and an outer surface configured to contact the wall of the target tissue region. In an exemplary configuration, as shown in FIG. 1, the expandable element 40 is a dual walled balloon, *i.e.,* a first inner balloon 42 inside of a second outer balloon 44, but, in some embodiments, may be a single walled balloon. Although not shown, in some embodiments, the expandable element 40 includes at least one pressure sensor coupled to the outer surface of the inner balloon 42 or the inner surface of the outer balloon 44 to continuously measure pressure within an interstitial space defined between the inner and outer balloons 42, 44. The measured pressure within the interstitial space can be transmitted to the console 12 and analyzed to determine the likelihood of a single or double-balloon breach of the inner balloon 42 and/or outer balloon 44.

Continuing to refer to FIG. 1, each of the supply lumen 36 and exhaust lumen may be in fluid communication with a fluid delivery conduit 46 which may wrap, spiral, or coil around a portion of the inner shaft 34. The fluid delivery conduit 46 may define a plurality of spray ports (e.g., openings, orifices, or apertures) and is configured to deliver cryogenic fluid through each port towards the inner surface of the expandable element 40. In some instances (for example, embodiments where the balloon 40 is a dual walled balloon) the ports are configured to deliver cryogenic fluid to the inner surface of the first inner balloon 42. In one configuration, one or more conductors (not shown) may be included within the one or more lumens and configured to relay signals to and from the medical device 24 to the processing circuitry 16 of the console 12.

Further, although not shown, the expandable element 40 may include a plurality of electrodes coupled to the inner surface and/or the outer surface of either the inner or outer balloons 42, 44. For example, in some configurations the plurality of electrodes are uniformly distributed on the outer surface of the outer balloon 44. In other configurations, the plurality of electrodes are unevenly distributed, for example, on a particular portion of the outer balloon 44. The plurality of electrodes may be configured to measure an inner surface temperature or outer surface temperature of the outer balloon 44, which may be indicative of the inner surface temperature or outer surface temperature of the expandable element 40 as a whole. For example, at least one of the plurality of electrodes may be a thermocouple configured to measure the temperature of the inner balloon 42 and/or outer balloon 44 before, during, and/or after the delivery of refrigerant to the expandable element 40. The measurement of the temperature of the expandable element 40 may be used, in part, to determine the amount or degree of ice formation at the ablation site.

Further, as shown in FIG. 1, the medical device 24 may include a pressure monitoring tube 48. The pressure monitoring tube 48 may extend through the elongate body 26 parallel to the inner shaft 34 and is in electrical and/or fluid communication with the console 12. As shown in FIG. 1, a distal end portion 50 of the pressure monitoring tube 48 is disposed within the expandable element 40 and is configured to periodically or continuously measure the internal pressure of the expandable element 40, including the internal pressure of the first inner balloon 42. As the pressure monitoring tube 48 measures or records the internal pressure of the expandable element 40, the monitoring tube 48 transmits a signal to the console 12 that is indicative of the measured pressure. The processing circuitry 16 of the console 12 may then process the received signal and perform or execute one or more actions based on the measured pressure. For example, in some configurations, if the measured internal pressure of the expandable element 40 exceeds a predetermined threshold, the processing circuitry 16 may trigger the stoppage of fluid delivery to the expandable element 40 during either the inflation phase or the ablation phase. It is to be understand that as used herein, "inflation phase" refers to a first phase of a treatment procedure in which the expandable element 40 is inflated prior to treating tissue with thermal energy (i.e., the "ablation phase"). Stopping the delivery of fluid to the expandable element 40 may be performed as a safety measure that may help prevent a breach of the expandable element 40 that could be harmful to the patient undergoing the treatment procedure.

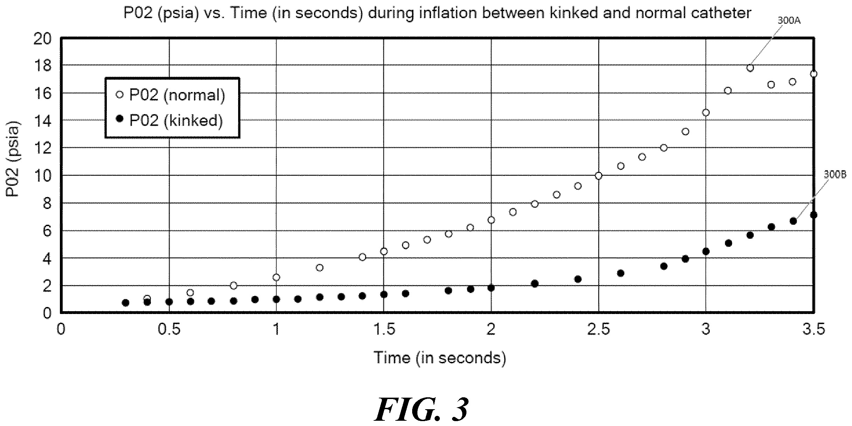

Continuing to refer to FIGS. 1-3, the console 12 includes at least one pressure sensor 52 and a vacuum pump 54. The vacuum pump 54 in the console 12 creates a low pressure environment in one or more lumens within the elongate body 26 so that cryogenic fluid is drawn from the exhaust lumen, away from the expandable element 40, towards the proximal portion 30 of the elongate body 26, and into an exhaust chamber 58 within the console 12. As shown in FIG. 2, a fluid flow path is defined within the console 12, and between the device 24 and the fluid supply reservoir 14 and exhaust chamber 58. In some embodiments, the pressure sensors 52 may be disposed within the console 12 to continuously measure a pressure within vacuum return path 61 (e.g., exhaust line) and/or measuring a period of time it takes for the pressure within the vacuum return path 61 to reach a target pressure or target pressure threshold. The pressure sensors 52 may then generate and transmit a pressure signal to the processing circuitry 16 of the console 12 that is indicative of the time it takes for the pressure within the vacuum return path 61 to reach the target pressure. If the pressure within the fluid vacuum return path 61 does not reach the target pressure within a predetermined time period, the processing circuitry 16 triggers a fault or command to close the proportional valve 60 (PV1) and/or the safety solenoid valve 62, which stops the delivery of fluid to the expandable element 40. For example, as shown in FIG. 3, the vacuum return path 61 of a catheter under normal operating conditions (indicated by line 300A) is capable of reaching the target pressure of at least 3 psia within 1 seconds from the time the injection of fluid into the expandable element is initiated, whereas the vacuum return path 61 of a catheter experiencing an adverse event (discussed in more detail below), may take longer than 2 seconds to reach 3 psia (indicated by line 300B).

Once the processing circuitry 16 has closed PV1 60 and/or the safety solenoid valve 62 to stop the delivery of fluid to the expandable element 40, the processing circuitry 16 may then generate at least one of an audible, visual, and/or tactile alert that is indicative of the presence of an adverse event and transmit the alert to an external control unit, the display 22, and/or a smart device (e.g., tablet device) of the clinician. In some embodiments, the adverse event may be a mechanical condition that restricts or lessens the flow of fluid through elongate body 26 to the vacuum return path 61. For example, the adverse event may be the presence of a kink/bend in the elongate body 26 (for example, including the fluid exhaust lumen) while fluid is being delivered to the expandable element 40. Additionally, in other embodiments, the adverse event may be the detection of blockages in the supply lumen 36 or fluid delivery conduit 46 during the delivery of fluid to the expandable element 40. The blockages may be the result of debris or ice formation that partially or fully blocks or clogs the supply lumen 36 and/or fluid delivery conduit 46 and restricts the passage of fluid to the interior of the expandable element 40. In some embodiments, the adverse event may also include the detection of blockages in a fluid injection path 63 within the console 12. Although blockages in the fluid injection path 63 within the console 12 may not lead to a single balloon or double-balloon breach, it may be useful to alert clinicians to other adverse conditions prevalent in the system 10 during a medical procedure.

Now referring to FIGS. 2-4, in some embodiments the system 10 also includes a flow sensor 64 disposed within the vacuum return path 61. In some configurations, the flow sensor 64 is configured to measure a mass flow rate of fluid traveling from the expandable element 40 to the fluid exhaust chamber 58 via the vacuum return path 61 during the ablation phase (i.e., cooling of the expandable element 40 after it has been inflated to a target pressure). However, it is to be understood that the flow sensor may also be used to measure the mass flow rate of fluid during the inflation of the expandable element (the "inflation phase"). The processing circuitry 16 of the console 12 is configured to continuously compare the measured mass flow rate of fluid to a predetermined mass flow rate threshold, and trigger the closing of PV1 60 and/or the safety solenoid valve 62 to stop the delivery of fluid to the expandable element 40 if the measured mass flow rate of fluid does not reach the mass flow rate threshold or deviates from the mass flow rate threshold by more than a predetermined range.

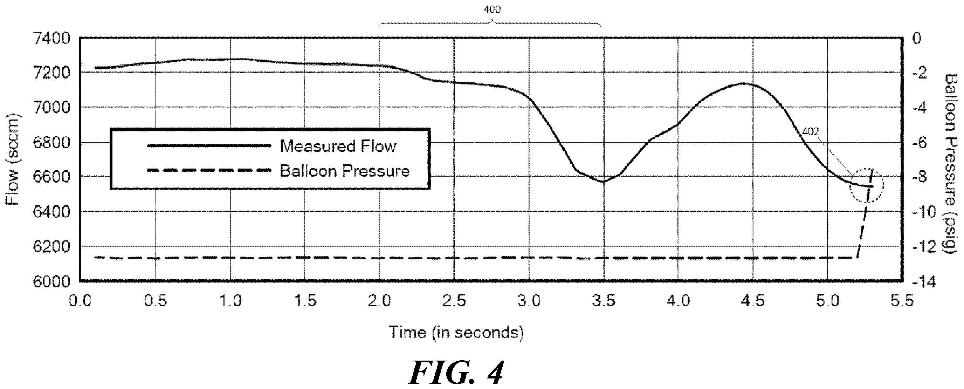

For example, as shown in FIG. 4, in some configurations the predetermined mass flow rate threshold is 7200 standard cubic centimeters per minute (sccm). When the measured flow dips below 7200 sccm by approximately 400-500 sccm, the processing circuitry 16 may determine that the device 24 is experiencing the adverse event. The clinician accordingly may be notified (for example, via the alert generated by the processing circuitry 16) in response to the detection of the presence of the adverse event prior to a breach of the inner balloon 42 and/or outer balloon 44 in a double-balloon embodiment of the expandable element 40. For example, the dip 400 from about 7200 sccm to about 6600 sccm in FIG. 4 can be detected around 3.5 seconds from the time of initiating the injection of fluid to the expandable element 40, whereas a breach of the inner and/or outer balloons 42, 44, of the expandable element 40 occurs around 5.2 seconds (indicated by circle 402). The dip 400 starting around 7200 may be due to a kink in the elongate body 26 soon before the time of the dip 400. Although the kinking of the elongate body does not restrict the delivery of fluid through the fluid supply lumen 36 (and thus does not lead to the buildup of pressure in the fluid injection path 63), the kinking of the elongate body 26 makes it more difficult for fluid to be exhausted from the expandable element 40 through the exhaust lumen, which leads to the buildup of pressure in the inner and/or outer balloons 42, 44, of the expandable element 40. Although it has been described herein that the dip 400 can be detected around 3.5 seconds from the time of initiating the injection of fluid to the expandable element 40, it is to be understood that 3.5 seconds is merely an example time and the dip 400 may instead be detected anytime during ablation once the fluid flow rate has reached 7200 sccm, when a kink has occurred.

Because the dip 400 in measured flow can be detected sooner than a sudden or rapid increase in balloon pressure, the console 12 is configured to detect the presence of the adverse event at an earlier point in time during the treatment procedure. The console 12 includes software and/or hardware to trigger a fault or other type of executable command to stop the injection or delivery of fluid to the expandable element 40 prior to the expandable element 40 experiencing a breach. Because manual stoppage of fluid delivery by clinicians may not be fast enough, the console 12 is configured to automatically stop the injection of fluid to the expandable element 40 once it has detected the adverse event.

As shown in FIG. 4, the inner and/outer balloon 44 of the expandable element 40 experiences a sharp spike in pressure at about 5.2 seconds, which may indicate a sudden breach or bursting of the inner and/or outer balloons 42, 44. As described previously regarding FIG. 3, once the processing circuitry 16 has triggered the closing of PV1 60 and/or the safety solenoid valve 62, stopping the delivery of fluid to the expandable element 40, the processing circuitry 16 may then generate and transmit an alert that is indicative of the presence of an adverse event. As also previously described above, the adverse event may be a mechanical condition that restricts or lessens the flow of fluid through the vacuum return path 61.

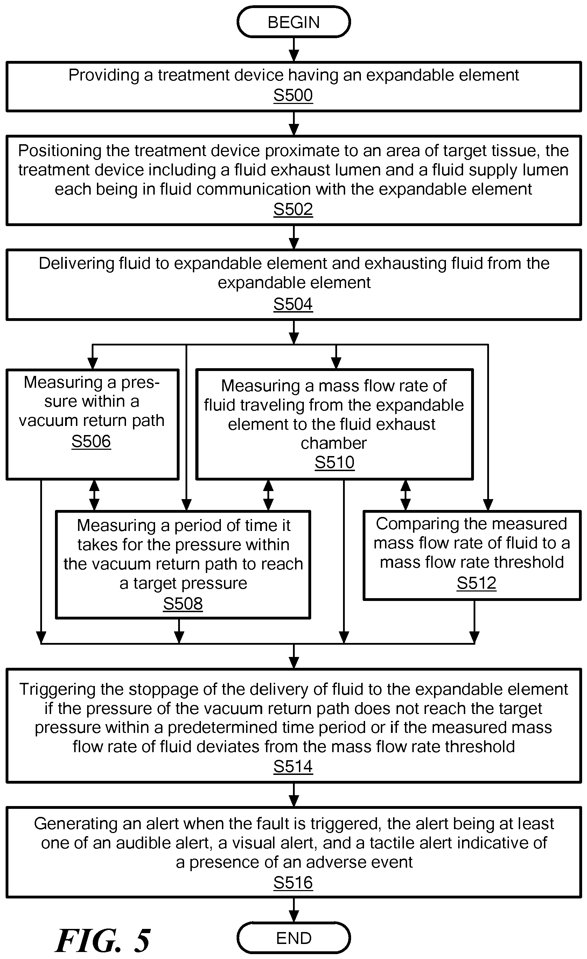

FIG. 5is a flowchart illustrating a method of predicting an adverse event during a medical procedure according to some embodiments. The method includes providing a medical device 24 having the expandable element 40, and the console 12 in communication with the medical device 24 (Block S500). As mentioned above, the console 12 includes processing circuitry 16, fluid supply reservoir 14, and the fluid exhaust chamber 58 disposed therein. The device 24 is then advanced through the vasculature of the patient and positioned proximate to an area of target tissue (Block S502). The processing circuitry 16 of the console 12 may then initiate the delivery of fluid from the fluid supply reservoir 14 to the expandable element 40 and the periodic or continuous exhausting of fluid from the expandable element 40 to the fluid exhaust chamber 58 (Block S504). As fluid is delivered to the expandable element 40, the pressure sensor 52 is configured to measure a pressure of the vacuum return path 61 and transmit a signal indicative of the measured pressure to the processing circuitry 16 (Block S506). The processing circuitry 16 is configured to measure and determine a period of time it takes for the measured pressure to reach a target pressure (Block S508). Additionally and/or alternatively, during either the inflation and/or ablation phases, a flow sensor 64 disposed within the vacuum return path 61 is configured to measure the mass flow rate of fluid traveling from the expandable element 40 to the fluid exhaust chamber 58 via the vacuum return path 61 (Block S510), and the processing circuitry 16 then compares the measured mass flow rate of fluid to a mass flow rate threshold (Block S512). The processing circuitry 16 may then stop the delivery of fluid to the expandable element 40 if the pressure of the vacuum return path 61 does not reach the target pressure within a predetermined time period and/or if the measured mass flow rate of fluid deviates from the mass flow rate threshold by closing PV1 60 and/or the safety solenoid valve 62 (Block S514). Once the delivery of fluid has been stopped by the processing circuitry 16, an audible, visual, and/or tactile alert indicative of a presence of an adverse event is generated by the processing circuitry 16 and may be transmitted to an external control unit, the display 22, and/or a smart device (Block S516).

It should be understood that various aspects disclosed herein may be combined in different combinations than the combinations specifically presented in the description and accompanying drawings. It should also be understood that, depending on the example, certain acts or events of any of the processes or methods described herein may be performed in a different sequence, may be added, merged, or left out altogether (e.g., all described acts or events may not be necessary to carry out the techniques). In addition, while certain aspects of this disclosure are described as being performed by a single module or unit for purposes of clarity, it should be understood that the techniques of this disclosure may be performed by a combination of units or modules associated with, for example, a medical device.

In one or more examples, the described techniques may be implemented in hardware, software, firmware, or any combination thereof. If implemented in software, the functions may be stored as one or more instructions or code on a computer-readable medium and executed by a hardware-based processing unit. Computer-readable media may include non-transitory computer-readable media, which corresponds to a tangible medium such as data storage media (e.g., RAM, ROM, EEPROM, flash memory, or any other medium that can be used to store desired program code in the form of instructions or data structures and that can be accessed by a computer).

Instructions may be executed by one or more processors, such as one or more digital signal processors (DSPs), general purpose microprocessors, application specific integrated circuits (ASICs), field programmable logic arrays (FPGAs), or other equivalent integrated or discrete logic circuitry. Accordingly, the term "processor" as used herein may refer to any of the foregoing structure or any other physical structure suitable for implementation of the described techniques. Also, the techniques could be fully implemented in one or more circuits or logic elements.

It will be appreciated by persons skilled in the art that the present invention is not limited to what has been particularly shown and described herein above. In addition, unless mention was made above to the contrary, it should be noted that all of the accompanying drawings are not to scale. A variety of modifications and variations are possible in light of the above teachings without departing from the scope and spirit of the invention, which is limited only by the following claims.

A method of predicting an adverse event during an ablation procedure includes providing a medical device having an expandable element and positioning the medical device proximate to an area of target tissue. The medical device includes a fluid exhaust lumen and a fluid supply lumen each being in fluid communication with the expandable element. The method further includes delivering fluid to expandable element and exhausting fluid from the expandable element; measuring a pressure within a vacuum return path; and measuring a period of time it takes for the pressure within the vacuum return path to reach a target pressure.

Further disclosed herein is the subject-matter of the following clauses:
1. A method of predicting an adverse event during an ablation procedure, the method comprising:
   providing a medical device having an expandable element;
   positioning the medical device proximate to an area of target tissue, the medical device including a fluid exhaust lumen and a fluid supply lumen each being in fluid communication with the expandable element;
   delivering fluid to expandable element and exhausting fluid from the expandable element;
   measuring a pressure within a vacuum return path; and
   measuring a period of time it takes for the pressure within the vacuum return path to reach a target pressure.
2. The method of clause 1, wherein the medical device is in communication with a console, the console including:
   processing circuitry;
   a fluid supply reservoir in fluid communication with the fluid supply lumen; and
   a fluid exhaust chamber in fluid communication with the fluid exhaust lumen.
3. The method of clause 1 or 2, further including:
   triggering a fault to stop the delivery of fluid if the pressure within the vacuum return path does not reach the target pressure within a predetermined time period.
4. The method of clause 3 or of any of the preceding clauses, wherein the vacuum return path is disposed within the console, the pressure of the vacuum return path is measured by a pressure sensor disposed within the console along the vacuum return path.
5. The method of clause 4 or of any of the preceding clauses, further including:
   generating an alert when the fault to stop the delivery of fluid is triggered, the alert being at least one of an audible alert, a visual alert, and a tactile alert indicative of a presence of an adverse event.
6. The method of clause 5 or of any of the preceding clauses, wherein the adverse event is a condition causing the restriction of fluid through the vacuum return path.
7. The method of clause 6 or of any of the preceding clauses, further including:
   measuring, with a flow sensor, a mass flow rate of fluid traveling from the expandable element to the fluid exhaust chamber via the vacuum return path.
8. The method of clause 7 or of any of the preceding clauses, further including:
   comparing the measured mass flow rate of fluid to a mass flow rate threshold.
9. The method of clause 8 or of any of the preceding clauses, further including:
   determining, based on the comparison of the measured mass flow rate of fluid to the mass flow rate threshold, whether the adverse event is present;
   triggering the stoppage of fluid delivery it is determined that the adverse event is detected based on the comparison of the measured mass flow rate of fluid to the mass flow rate threshold; and
   generating the alert when the fault to stop the delivery of fluid is triggered.
10. The method of clause 1 or of any of the preceding clauses, wherein the medical device further includes a pressure monitoring tube at least partially disposed within the expandable element and configured to monitor an internal pressure of the expandable element.
11. A medical system, comprising:
   a console having processing circuitry;
   a medical device in communication with the console, the medical device including:
      an expandable element;
   a fluid supply lumen and a fluid exhaust lumen in fluid communication with the expandable element and the console, the fluid exhaust lumen being in communication with a vacuum return path disposed within the console; and
   a pressure sensor disposed within the console along the vacuum return path and configured to measure a pressure within the vacuum return path.
12. The system of clause 11, further including a fluid supply reservoir and a fluid exhaust chamber each disposed within the console and in fluid communication with the medical device.
13. The system of clause 11 or 12, wherein the processing circuitry is configured to measure a period of time it takes for the pressure within the fluid exhaust lumen to reach a target pressure.
14. The system of clause 13 or of any of the clauses 11 to 13, wherein the console is further configured to:
   initiate a delivery of fluid from the fluid supply reservoir to the expandable element; and
   trigger the stoppage of the delivery of fluid to the expandable element if the measured pressure within the vacuum return path does not reach the target pressure within a predetermined time period.
15. The system of clause 14 or of any of the clauses 11 to 14, wherein the console is configured to generate and transmit an alert when the fault is triggered, the alert being at least one of an audible alert, a visual alert, and a tactile alert indicative of a presence of an adverse event.
16. The system of clause 15 or of any of the clauses 11 to 15, further including a flow sensor disposed within the console along the vacuum return path, the flow sensor being configured to measure a mass flow rate of fluid traveling from the expandable element to the fluid exhaust chamber via the vacuum return path.
17. The system of clause 16 or of any of the clauses 11 to 16, wherein the processing circuitry is configured to:
   compare the measured mass flow rate of fluid to a mass flow rate threshold; and
   trigger the stoppage of the delivery of fluid to the expandable element if the measured mass flow rate of fluid deviates from the mass flow rate threshold by more than a predetermined range.
18. The system of clause 11 or of any of the clauses 11 to 17, wherein the medical device further includes a pressure monitoring tube at least partially disposed within the expandable element and configured to monitor an internal pressure of the expandable element.
19. A method of predicting an adverse event during an ablation procedure, the method comprising:
   providing a medical device having an expandable element, the medical device being in communication with a console having:
      processing circuitry;
      a fluid supply reservoir;
      a fluid exhaust chamber; and
      a vacuum return path;
   positioning the medical device proximate to an area of target tissue, the medical device including a fluid exhaust lumen and a fluid supply lumen each being in fluid communication with the expandable element and the console;
   delivering fluid from the fluid supply reservoir to expandable element and exhausting fluid from the expandable element to the fluid exhaust chamber;
   at least one of:
      (a) measuring a pressure of the vacuum return path, the pressure being measured by a pressure sensor disposed within the console along the vacuum return path;
      (b) measuring a period of time it takes for the pressure within the vacuum return path to reach a target pressure;
      (c) measuring, with a flow sensor, a mass flow rate of fluid traveling from the expandable element to the fluid exhaust chamber via the vacuum return path; and
      (d) comparing the measured mass flow rate of fluid to a mass flow rate threshold; and
   triggering the stoppage of the delivery of fluid to the expandable element if the pressure of the vacuum return path does not reach the target pressure within a predetermined time period or if the measured mass flow rate of fluid deviates from the mass flow rate threshold by more than a predetermined range.
20. The method of clause 19, further including:
   generating an alert when the fault is triggered, the alert being at least one of an audible alert, a visual alert, and a tactile alert indicative of a presence of an adverse event.

## Claims

1. A method of predicting an adverse event during an ablation procedure, the method comprising:
providing a medical device having an expandable element;
positioning the medical device proximate to an area of target tissue, the medical device including a fluid exhaust lumen and a fluid supply lumen each being in fluid communication with the expandable element;
delivering fluid to expandable element and exhausting fluid from the expandable element;
measuring a pressure within a vacuum return path; and
measuring a period of time it takes for the pressure within the vacuum return path to reach a target pressure.

2. The method of Claim 1, wherein the medical device is in communication with a console, the console including:
processing circuitry;
a fluid supply reservoir in fluid communication with the fluid supply lumen; and
a fluid exhaust chamber in fluid communication with the fluid exhaust lumen,
and/or
wherein the medical device further includes a pressure monitoring tube at least partially disposed within the expandable element and configured to monitor an internal pressure of the expandable element.

3. The method of Claim 1 or 2, further including:
triggering a fault to stop the delivery of fluid if the pressure within the vacuum return path does not reach the target pressure within a predetermined time period.

4. The method of one of the Claims 1 to 3, wherein the vacuum return path is disposed within the console, the pressure of the vacuum return path is measured by a pressure sensor disposed within the console along the vacuum return path.

5. The method of one of the Claims 1 to 4, further including:
generating an alert when the fault to stop the delivery of fluid is triggered, the alert being at least one of an audible alert, a visual alert, and a tactile alert indicative of a presence of an adverse event.

6. The method of one of the Claims 1 to 5, wherein the adverse event is a condition causing the restriction of fluid through the vacuum return path.

7. The method of one of the Claims 1 to 6, further including:
measuring, with a flow sensor, a mass flow rate of fluid traveling from the expandable element to the fluid exhaust chamber via the vacuum return path,
particularly
further including:
comparing the measured mass flow rate of fluid to a mass flow rate threshold,
particularly
further including:
determining, based on the comparison of the measured mass flow rate of fluid to the mass flow rate threshold, whether the adverse event is present;
triggering the stoppage of fluid delivery it is determined that the adverse event is detected based on the comparison of the measured mass flow rate of fluid to the mass flow rate threshold; and
generating the alert when the fault to stop the delivery of fluid is triggered.

8. A medical system, comprising:
a console having processing circuitry;
a medical device in communication with the console, the medical device including:
an expandable element;
a fluid supply lumen and a fluid exhaust lumen in fluid communication with the expandable element and the console, the fluid exhaust lumen being in communication with a vacuum return path disposed within the console; and
a pressure sensor disposed within the console along the vacuum return path and configured to measure a pressure within the vacuum return path.

9. The system of Claim 8, further including a fluid supply reservoir and a fluid exhaust chamber each disposed within the console and in fluid communication with the medical device, and/or
wherein the medical device further includes a pressure monitoring tube at least partially disposed within the expandable element and configured to monitor an internal pressure of the expandable element.

10. The system of Claim 8 or 9, wherein the processing circuitry is configured to measure a period of time it takes for the pressure within the fluid exhaust lumen to reach a target pressure.

11. The system of one of the Claims 8 to 10, wherein the console is further configured to:
initiate a delivery of fluid from the fluid supply reservoir to the expandable element; and
trigger the stoppage of the delivery of fluid to the expandable element if the measured pressure within the vacuum return path does not reach the target pressure within a predetermined time period.

12. The system of one of the Claims 8 to 11, wherein the console is configured to generate and transmit an alert when the fault is triggered, the alert being at least one of an audible alert, a visual alert, and a tactile alert indicative of a presence of an adverse event.

13. The system of one of the Claims 8 to 12, further including a flow sensor disposed within the console along the vacuum return path, the flow sensor being configured to measure a mass flow rate of fluid traveling from the expandable element to the fluid exhaust chamber via the vacuum return path.

14. The system of one of the Claims 8 to 13, wherein the processing circuitry is configured to:
compare the measured mass flow rate of fluid to a mass flow rate threshold; and
trigger the stoppage of the delivery of fluid to the expandable element if the measured mass flow rate of fluid deviates from the mass flow rate threshold by more than a predetermined range.

15. A method of predicting an adverse event during an ablation procedure, the method comprising:
providing a medical device having an expandable element, the medical device being in communication with a console having:
processing circuitry;
a fluid supply reservoir;
a fluid exhaust chamber; and
a vacuum return path;
positioning the medical device proximate to an area of target tissue, the medical device including a fluid exhaust lumen and a fluid supply lumen each being in fluid communication with the expandable element and the console;
delivering fluid from the fluid supply reservoir to expandable element and exhausting fluid from the expandable element to the fluid exhaust chamber;
at least one of:
(a) measuring a pressure of the vacuum return path, the pressure being measured by a pressure sensor disposed within the console along the vacuum return path;
(b) measuring a period of time it takes for the pressure within the vacuum return path to reach a target pressure;
(c) measuring, with a flow sensor, a mass flow rate of fluid traveling from the expandable element to the fluid exhaust chamber via the vacuum return path; and
(d) comparing the measured mass flow rate of fluid to a mass flow rate threshold; and
triggering the stoppage of the delivery of fluid to the expandable element if the pressure of the vacuum return path does not reach the target pressure within a predetermined time period or if the measured mass flow rate of fluid deviates from the mass flow rate threshold by more than a predetermined range,
particularly further including:
generating an alert when the fault is triggered, the alert being at least one of an audible alert, a visual alert, and a tactile alert indicative of a presence of an adverse event.
